# EUROPÄISCHE PATENTANMELDUNG

(11) **EP 1 719 752 A1**
(43) Veröffentlichungstag der Anmeldung: **08.11.2006**
(21) Anmeldenummer: 06007635.3
(22) Anmeldetag: 12.04.2006
(51) Int. Cl.: C07C 45/50, C07C 47/277, C07D 317/54

(54) **Verfahren zur Hydroformylierung von substituierten Allylbenzolen**

(30) Priorität: 26.04.2005 DE 102005019237
(71) Anmelder: Celanese Chemicals Europe GmbH, 61476 Kronberg (DE)
(72) Erfinder: Springer, Helmut, 46539 Dinslaken (DE); Rudner, Paul, 46147 Oberhausen (DE)

(57) **Zusammenfassung**

Die vorliegende Erfindung betrifft ein Verfahren zur Hydroformylierung von substituierten Allylbenzolen, insbesondere von Eugenol, Estragol, Eugenolmethylether und Safrol in Gegenwart einer wässrigen Katalysatorlösung, enthaltend wasserlösliche Rhodiumverbindungen, die sulfonierte Arylphosphine in komplexer Bindung enthalten.

## Beschreibung

Die vorliegende Erfindung betrifft ein Verfahren zur Hydroformylierung von substituierten Allylbenzolen in Gegenwart einer wässrigen Katalysatorlösung, in der wasserlösliche, sulfonierte Arylphosphine in komplexer Bindung enthaltende Rhodiumverbindungen gelöst vorliegen.

Es ist bekannt, olefinisch ungesättigte Verbindungen mit einem Gemisch aus Kohlenmonoxid und Wasserstoff in Gegenwart katalytisch wirkender Metallverbindungen zu Aldehyden umzusetzen. Diese als Hydroformylierungsreaktion oder auch als Oxosynthese bezeichnete Reaktion wird nicht nur bei der Umsetzung einfacher Olefine, wie Propen oder Buten, in großem Maße angewandt sondern auch bei der Umsetzung komplexerer olefinischer Ausgangsverbindungen. Die Hydroformylierungsreaktion führt zu geradkettigen n-Aldehyden sowie zu verzweigten iso-Aldehyden und die Selektivität zu dem jeweiligen Aldehyd kann durch die Reaktionsbedingungen gesteuert werden.

So liefert die Hydroformylierungsreaktion von substituierten Allylbenzolen 3-Phenylpropanale und 4-Phenylbutanale, die angenehme Riechstoffeigenschaften besitzen und als wertvolle Zwischenprodukte unter Nutzung der Reaktivität der Aldehydgruppe weiterverarbeitet werden können. So lassen sich die Aldehyde nach bekannten Verfahren, zum Beispiel mittels einer aminierenden Hydrierung in Amine überführen, die wiederum zur Herstellung von Farbstoffen oder Pflanzenschutzmitteln eingesetzt werden.

Die Hydroformylierung von substituierten Allylbenzolen, wie 1-Hydroxy-2-methoxy-4-allylbenzol, auch als Eugenol bekannt oder von 1,2-Methylendioxy-4-allylbenzol, auch als Safrol bezeichnet, wird in DE-A1-2 235 466 beschrieben. Die Umsetzung erfolgt in Gegenwart von Rhodiumcarbonylkomplexen, wobei die als Ausgangsstoffe verwendeten Verbindungen und deren Hydroformylierungsprodukte als Lösungsmittel für die Rhodiumkomplexe dienen. Zweckmäßigerweise setzt man jedoch ein organisches Lösungsmittel, zum Beispiel Kohlenwasserstoffe, Ether oder Alkohole hinzu. Bei dem Verfahren nach DE-A1-2 235 466 beobachtet man bei der olefinischen Ausgangsverbindung eine Isomerierung der endständigen Doppelbindung in eine innenständige Position und die Hydroformylierung des Isomerisierungsproduktes führt zu Verbindungen mit einer Formylgruppe an dem Kohlenstoffatom, das direkt an dem aromatischen Ring gebunden ist. Diese Isomerisierungsprodukte können auch als Acetaldehydderivate aufgefasst werden. Die Bildung dieser unerwünschten isomeren Verbindung wird besonders bei erhöhter Hydroformylierungstemperatur begünstigt. DE-A1-2 235 466 empfiehlt daher eine vergleichsweise geringe Reaktionstemperatur von 70°C, bei der sich der geradkettige n-Aldehyd, d.h. das entsprechend substituierte 4-Phenylbutanal und der verzweigte iso-Aldehyd, d.h. das entsprechend substituierte 3-Phenylpropanal, in vergleichbarer Menge bilden. Zur Isolierung der reinen Aldehyde aus diesem Gemisch schlägt DE-A1-2 235 466 den Zusatz alkalischer Reagenzien vor, wodurch nur der zur Dimerisierung fähige geradkettige Aldehyd in eine höher siedende Verbindung überführt wird, während der unveränderte iso-Aldehyd als Reinsubstanz aus dem Gemisch destillativ abgetrennt werden kann. Der geradkettige Aldehyd kann aus dem Gemisch in der Weise gewonnen werden, dass man ihn mit einer katalytischen Menge Säure in die trimere Verbindung überführt, abtrennt und abschließend mit Säure in der Hitze wieder depolymerisiert.

Untersuchungen zur Hydroformylierung von substituierten Allylbenzolen, die insbesondere auf die Verbesserung der Regioselektivität in Richtung geradkettiger Aldehyde abzielen, sind ebenfalls Gegenstand der wissenschaftlichen Literatur.

So beschreibt Nouveau Journal De Chemie, Vol. 8, Nr. 4, 1984, Seiten 213-216 die Hydroformylierung von Estragol (1-Methoxy-4-allylbenzol), Eugenol, Safrol und Eugenolmethylether (1, 2-Dimethoxy-4-allylbenzol) in homogener Lösung unter Verwendung von [RhH(CO)(PPh₃)₃] und des zweikernigen Rhodiumkomplexes [Rh₂(µ-S-t-Bu)₂(CO)₂(P(OMe)₃)₂]. Bei der Verwendung der Phosphin modifizierten Rhodiumkomplexe wird nur eine geringe Isomerisierung der endständigen Doppelbindung in die innenständige Position beobachtet. Der einkernige Rhodiumkomplex zeigt zwar eine gute Anfangsaktivität, die jedoch aufgrund der Bildung inaktiver höherkerniger Rhodiumverbindungen rasch abnimmt. Die ebenfalls untersuchte zweikernige Rhodiumverbindung besitzt zwar eine erhöhte Aktivität, insbesondere bei der Hydroformylierung von Estragol, doch wird bei beiden untersuchten Katalysatorsystemen ein Verhältnis von n- zu iso-Aldehyd im Bereich von 80:20 bis 70:30 beobachtet.

Der Einfluss der Phosphorkomponente in dem zweikernigen Rhodiumkomplex [Rh₂(µ-S-t-Bu)₂(CO)₂(P(OMe)₃)₂] auf das n- /iso-Verhältnis bei der Hydroformylierung von Estragol, Eugenol, Safrol und Eugenolmethylether ist Gegenstand von Journal of Molecular Catalysis, 36 (1986) 349-357. Bei den ebenfalls in einem homogenen Reaktionsmedium durchgeführten Umsetzungen kann die Ausbeute an dem jeweiligen linearen Aldehyd erhöht werden, wenn man als Phosphorkomponente in dem zweikernigen Rhodiumkomplex [Rh₂(µ-S-t-Bu)₂(CO)₂(P(OMe)₃)₂] einen Phosphitliganden wie P(OMe)₃ oder P(OPh)₃ wählt. Bei dem am stärksten basischen Liganden PPh₃ als Phosphorkomponente in dem zweikernigen Rhodiumkomplex erhält man durchweg höhere Gehalte an dem entsprechenden iso-Aldehyd.

Journal of Molecular Catalysis A: Chemical 179 (2002), Seite 133-141 untersucht neben dem Einfluss von einzähnigen Phosphinliganden auch den Effekt von zweizähnigen Phosphinliganden bei der Rhodium katalysierten Hydroformylierung von Eugenol, Estragol, Eugenolmethylether und Safrol. Die Umsetzungen erfolgen in einem organischen Lösungsmittel unter Verwendung des zweikernigen Rhodiumkomplexes [Rh(COD)(OAc)]₂ (COD bedeutet 1,5 Cyclooctadien) als Katalysatorvorstufe in Gegenwart von Phosphinen und Diphosphinen, wie 1,2-Bis(diphenylphosphino)ethan, 2,2'-Bis[(diphenylphosphino)methyl]-1,1'-binaphthyl oder 2,2'-Bis[(diphenylphosphino)methyl-1,1'-biphenyl. Bei der Verwendung von Diphosphinen findet die Isomerisierung im eingesetzten Allylbenzol unter Wanderung der allylischen Doppelbindung sowie die Hydrierung der Doppelbindung in nur geringem Ausmaße statt. Das Verhältnis von n- zu iso-Aldehyd wird jedoch drastisch von der Natur des eingesetzten Diphosphins bestimmt. Während beim Einsatz von 1,2-Bis(diphenylphosphino)ethan der iso-Aldehyd bevorzugt gebildet wird, überwiegt bei der Verwendung von 2,2'-Bis[(diphenylphosphino)methyl]-1,1'-binaphthyl als Phosphinligand der geradkettige Aldehyd. Mit 2,2'-Bis[(diphenylphosphino)methyl]-1,1'-binaphthyl in der Hydroformylierungsreaktion erhält man gute Ausbeuten an den gewünschten linearen Aldehyden. Nachteilig ist jedoch der Einsatz komplexer Phosphinliganden, die sich nur aufwendig synthetisieren lassen. Ebenfalls erfordert die destillative Abtrennung der gewünschten Aldehyde aus dem homogenen organischen Reaktionsmedium eine thermische Belastung des Reaktionsguts, die zu einer Schädigung des Katalysators und des Diphosphin-Liganden führen kann. Daher wird der Wiedereinsatz des nach Aldehydabtrennung anfallenden Katalysator haltigen Rückstands in die Hydroformylierungsreaktion erschwert, was wiederum mit ökologischen und wirtschaftlichen Nachteilen verbunden ist.

Es ist ebenfalls bekannt, Hydroformylierungsreaktionen in einem heterogenen Zweiphasensystem durchzuführen. Ein solcher Prozess ist charakterisiert durch das Vorliegen einer organischen Phase, die das Ausgangsolefin und das Reaktionsprodukt enthält, und einer wässrigen Phase, in der der Katalysator gelöst ist. Als Katalysatoren finden wasserlösliche Rhodiumkomplexverbindungen mit wasserlöslichen Phosphinen als Liganden Anwendung. Zu den wasserlöslichen Phosphinen zählen insbesondere Triarylphosphine, Trialkylphosphine oder Alkylaryldiphosphine, deren organische Reste durch Sulfonsäuregruppen oder Carboxylgruppen substituiert sind. Nach erfolgter Umsetzung können organische Phase und Katalysatorphase durch einfache Phasentrennung ohne thermische Belastung von einander getrennt werden und die wässrige Katalysatorphase kann in den Prozess zurückgeschleust werden. Diese Verfahrensvariante der Hydroformylierungsreaktion ist zum Beispiel Gegenstand der DE-A1-26 27 354.

Auch die Hydroformylierung von substituierten Allylbenzolen nach dem heterogenen Zweiphasenverfahren wird in der Literatur beschrieben. So behandelt New. J. Chem., 1988, 12, 687 die Hydroformylierung von Estragol, Sairol und Eugenolmethylether in Gegenwart einer wässrigen Lösung, die den zweikernigen Komplex Rh₂(µ-SR)₂(CO)₂(TPPTS)₂ gelöst enthält, wobei der Rest R beispielsweise eine tertiär-Butylgruppe bedeutet und TPPTS Tri(sulphophenyl)phosphin ist. Allerdings werden nur unbefriedigende Umsätze in die Aldehyde beobachtet. Es findet in einem bedeutenden Maße die Isomersierung der Doppelbindung in die für die Hydroformylierungsreaktion inaktive innenständige Position statt. Je nach eingesetztem Substrat ist die Linearität der gebildeten Aldehyde befriedigend bis gut.

Die bekannten Verfahren erfüllen in wirtschaftlicher und technischer Hinsicht noch nicht alle Anforderungen, die an einen im technischen Maßstab ausgeübten Prozess gestellt werden. So erfordern die bekannten homogenen Verfahren den Einsatz aufwendig zu synthetisierender Diphosphin-Liganden und die destillative Abtrennung der gewünschten Aldehyde aus dem Reaktionsgut mit der Gefahr der Katalysator- und Ligandenschädigung im Zuge der thermischen Belastung, was den Wiedereinsatz des Katalysators in die Hydroformylierungsreaktion erschwert. Hingegen wird bei den bekannten heterogenen Zweiphasenprozessen nur ein unbefriedigender Umsatz in die Aldehyde beobachtet.

Es bestand daher die Aufgabe, ein möglichst einfaches und kostengünstiges Verfahren zur Hydroformylierung von substituierten Allylbenzolen bereitzustellen, das bei hohem Umsatz an Olefin gleichzeitig den geradkettigen Aldehyd in hohen Mengen ergibt.

Die Erfindung besteht daher in einem Verfahren zur Hydroformylierung von substituierten Allylbenzolen. Es ist dadurch gekennzeichnet, dass man Allylbenzole der allgemeinen Formel (I) 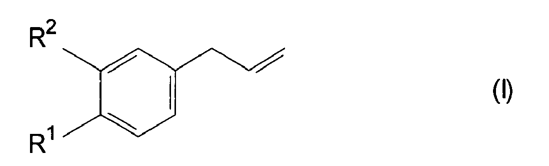 in der R¹ einen Hydroxyl- oder Alkoxyrest mit 1 bis 4 Kohlenstoffatomen bedeutet, R² für Wasserstoff, einen Hydroxyl- oder einen Alkoxyrest mit 1 bis 4 Kohlenstoffatomen steht, R¹ und R² gemeinsam eine Alkylendioxygruppe -[O-CH₂-O]ₙ- mit n gleich 1, 2 oder 3 bedeutet, in einem heterogenen Reaktionssystem unter Verwendung einer wässrigen Lösung von wasserlöslichen, sulfonierte Arylphosphine der allgemeinen Formel (II) 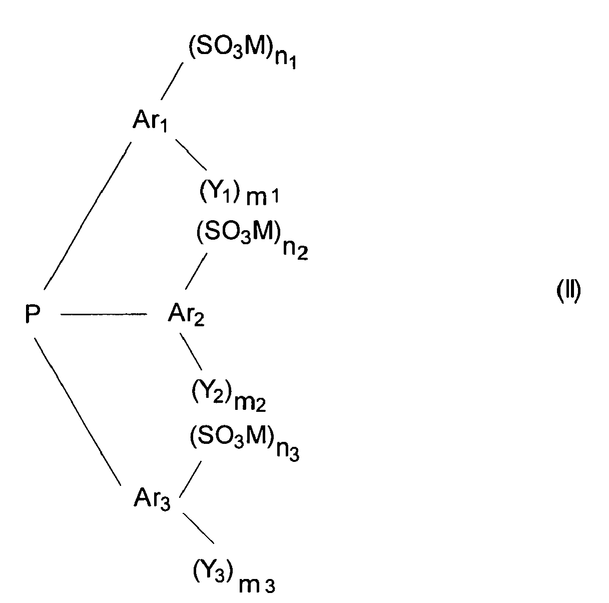 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR³R⁴ stehen, in der die Substituenten R³ und R⁴ gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist, in komplexer Bindung enthaltenden Rhodiumverbindungen bei Temperaturen von 80 bis 140°C und Drücken von 0,5 MPa bis 10 MPa mit Synthesegas umsetzt.

Überraschenderweise führt die Hydroformylierung in Gegenwart der sulfonierten Arylphosphine der allgemeinen Formel (II) selbst bei milden Temperatur- und Druckbedingungen nach dem heterogenen Zweiphasenverfahren zu einem nahezu vollständigen Olefinumsatz. Dies ist umso mehr überraschend, weil nach dem Stand der Technik, beispielsweise nach DE-A1-34 12 335 und DE-A1-31 35 127, die Hydroformylierung höherer Olefine nach dem heterogenen Zweiphasenverfahren den Zusatz eines Lösungsvermittlers erfordert, da höhere Olefine im Gegensatz zu den niedrigen Olefinen Propen und Buten nur eine sehr geringe Wasserlöslichkeit aufweisen.

Bei der Hydroformylierung der substituierten Allylbenzole der allgemeinen Formel (I) findet je nach Substituent in gewissem Maße eine Isomerisierung der endständigen allylischen Doppelbindung in die innenständige Position statt. Das durch die Wanderung der Doppelbindung gebildete innenständige Olefin verhält sich jedoch in dem heterogenen Zweiphasenprozess nahezu inert und kann aus dem erhaltenen Hydroformylierungsrohprodukt destillativ leicht abgetrennt werden. Der Gehalt des durch Isomerisierung gebildeten innenständigen Olefins im Hydroformylierungsrohprodukt liegt im allgemeinen zwischen 10 bis 20 Gew.-%.

Das erhaltene Hydroformylierungsrohprodukt enthält überwiegend den geradkettigen Aldehyd, nämlich das entsprechend substituierte 4-Phenylbutanal. Der verzweigte iso-Aldehyd, nämlich das entsprechend substituierte 3-Phenylpropanal, wird nur in untergeordneten Mengen gebildet und im allgemeinen beträgt das n-/iso-Verhältnis von 90:10 bis 95:5, je nach eingesetztem Substrat.

Zu den wasserlöslichen sulfonierten Arylphosphinen der allgemeinen Formel (II) zählen bevorzugt solche Arylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Die Bedingungen, unter denen die Umsetzung in der Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 80°C bis 140°C durch. Bevorzugt hält man Temperaturen von 100°C bis 130°C und insbesondere von 110°C bis 130°C ein. Der Gesamtdruck erstreckt sich über einen Bereich von 0,5 MPa bis 10 MPa, vorzugsweise 1 MPa bis 6 MPa und insbesondere 1,5 MPa bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Rhodium-Konzentration beträgt 20 bis 1000 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphor-Liganden, d.h. Ligand, der mit Rhodium keine komplexe Bindung eingegangen ist. Je mol Rhodium wendet man bevorzugt 10 bis 300 mol Phosphor in Form der wasserlöslichen, sulfonierten Arylphosphine der allgemeinen Formel (II) an. Besonders bewährt haben sich molare Verhältnisse von Rhodium zu Phosphor im Bereich von 1:50 bis 1:150. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Man bildet den Katalysator üblicherweise aus den Komponenten Rhodium oder Rhodiumverbindung, sulfoniertes Arylphosphin der allgemeinen Formel (II) und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Das substituierte Allybenzol der allgemeinen Formel (I) kann als solches oder in Lösung der Hydroformylierung zugeführt werden. Geeignete Lösungsmittel sind wasserunlösliche Ketone, Dialkylether, aliphatische Nitrile, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Hinsichtlich der verfahrenstechnischen und apparativen Ausgestaltung der Hydroformylierungsreaktion kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der Hydroformylierungsstufe wird in einem Phasentrenner in die organische Produktphase und in die wässrige Katalysatorlösung aufgetrennt. Es hat sich als zweckmäßig erwiesen, die Katalysatorlösung wieder in der Hydroformylierung einzusetzen. Zweckmäßigerweise wird sie im Kreis geführt. Die abgetrennte rohe organische Produktphase wird anschließend nach an sich bekannten Verfahren zu den gewünschten reinen Aldehyden aufgearbeitet, beispielsweise nach Destillationsverfahren.

Zur Herstellung des Hydroformylierungskatalysators gelangt Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Rhodiumoxide oder auch Rhodiumcarbonylverbindungen, wie Rh₃(CO)₁₂, Rh₆(CO)₁₆, oder Rhodium-Komplexverbindungen, z.B. Cyclopentadienylrhodiumverbindungen, Rhodiumacetylacetonat, oder [RhCl(Cyclooctadien-1,5]₂, eingesetzt werden. Rhodiumhalogenverbindungen kommen wegen ihres korrosiven Verhaltens der Halogenidionen weniger in Betracht.

Bevorzugt werden Rhodiumoxide und insbesondere Rhodiumacetat und -2-ethylhexanoat.

Die Hydroformylierungsreaktion kann sowohl absatzweise als auch kontinuierlich durchgeführt werden.

Das erfindungsgemäße Verfahren gestattet einen einfachen und kostengünstigen Zugang zu einem Gemisch aus 3-Phenylpropanalen und 4-Phenylbutanalen mit einem hohen Anteil an den geradkettigen 4-Phenylbutanalen. Das erfindungsgemäße Verfahren eignet sich besonders zur Hydroformylierung von Eugenol(1-Hydroxy-2-methoxy-4-allylbenzol), Estragol(1-Methoxy-4-allylbenzol) und Eugenolmethylether(1,2-Dimethoxy-4-allylbenzol).

Im folgenden wird das erfindungsgemäße Verfahren anhand einiger Beispiele näher erläutert, es ist jedoch nicht auf die beschriebenen Ausführungsformen beschränkt.

### Beispiele

### Beispiel 1: Hydroformylierung von 1-Hydroxy-2-methoxy-4-allylbenzol (Eugenol)

In einem 1 I-Autoklav wurden 223,0 g Wasser sowie 100,0 g TPPTS-Lösung mit einem P(III)-Gehalt von 629 mmol/kg vorgelegt und mit 11,5 g einer toluolischen Rh-2-ethylhexanoat-Lösung (Rh-Gehalt: 5650 mg/kg) versetzt. Danach wurde eine Mischung aus 141,2 g Eugenol und 60,5 g Toluol zugegeben. Das Reaktionsgemisch wurde auf 125°C erwärmt und bei einem Synthesegasdruck von 2,5 MPa und einer Reaktionszeit von 10 Stunden umgesetzt.

Nach Reaktionsende wurde abgekühlt und die obere, organische Phase von der wässrigen Katalysatorphase durch Phasentrennung abgetrennt.

Die organische Phase setzt sich wie folgt zusammen (Fl.-% lt. gaschromatographischer Analyse, der Anteil an Toluol ist nicht berücksichtigt):

| | |
|---|---|
| Vorlaufkomponenten | 0,2 % |
| Eugenol | <0,1 % |
| Komponenten | 0,9 % |
| Isoeugenol (1-Hydroxy-2-methoxy-4-propenyl-1-benzol) | 22,8 % |
| Komponenten | 0,4 % |
| 2-Ethyl-2-(3-methoxy-4-hydroxy-phenyl)-acetaldehyd | 4,1 % |
| 2-Methyl-3-(3-methoxy-4-hydroxy-phenyl)-propionaldehyd | 3,4 % |
| 4-(3-Methoxy-4-hydroxy-phenyl)-butanal | 62,7 % |
| Nachlaufkomponenten | 5,5 % |

Die im Autoklav verbleibende Katalysatorphase kann erneut mit einem Gemisch aus Eugenol und Toluol versetzt und zur Reaktion gebracht werden.

### Beispiel 2: Hydroformylierung von 1-Methoxy-4-allylbenzol (Estragol)

In einem 1 I-Autoklav wurden 223,0 g Wasser sowie 100,0 g TPPTS-Lösung mit einem P(III)-Gehalt von 629 mmol/kg vorgelegt und mit 11,5 g einer toluolischen Rh-2-ethylhexanoat-Lösung (Rh-Gehalt: 5650 mg/kg) versetzt. Danach wurde eine Mischung aus 127,5 g Estragol und 54,6 g Toluol zugegeben. Das Reaktionsgemisch wurde auf 125°C erwärmt und bei einem Synthesegasdruck von 2,5 MPa und einer Reaktionszeit von 10 Stunden umgesetzt.

Nach Reaktionsende wurde abgekühlt und die obere, organische Phase von der wässrigen Katalysatorphase durch Phasentrennung abgetrennt.

Die organische Phase setzt sich wie folgt zusammen (FI.-% lt. gaschromatographischer Analyse, der Anteil an Toluol ist nicht berücksichtigt):

| | |
|---|---|
| Vorlaufkomponenten | 1,2 % |
| Estragol | 2,5 % |
| Komponenten | 0,8 % |
| Anethol (1-Methoxy-4-propenyl-1-benzol) | 9,4 % |
| Komponenten | 0,2 % |
| 2-Ethyl-2-(4-methoxy-phenyl)-acetaldehyd | 0,2 % |
| 2-Methyl-3-(4-methoxy-phenyl)-propionaldehyd | 4,6 % |
| 4-(4-Methoxy-phenyl)-butanal | 79,0 % |
| Nachlaufkomponenten | 2,1 % |

Die im Autoklav verbleibende Katalysatorphase kann erneut mit einem Gemisch aus Estragol und Toluol versetzt und zur Reaktion gebracht werden.

### Beispiel 3: Hydroformylierung von 1,2-Dimethoxy-4-allylbenzol (Eugenolmethylether)

In einem 1 I-Autoklav wurden 223,0 g Wasser sowie 100,0 g TPPTS-Lösung mit einem P(III)-Gehalt von 629 mmol/kg vorgelegt und mit 9,2 g einer toluolischen Rh-2-ethylhexanoat-Lösung (Rh-Gehalt: 7062 mg/kg) versetzt. Danach wurde eine Mischung aus 153,3 g Eugenolmethylether und 65,7 g Toluol zugegeben. Das Reaktionsgemisch wurde auf 125°C erwärmt und bei einem Synthesegasdruck von 2,5 MPa und einer Reaktionszeit von 4 Stunden umgesetzt.

Nach Reaktionsende wurde abgekühlt und die obere, organische Phase von der wässrigen Katalysatorphase durch Phasentrennung abgetrennt.

Die organische Phase setzt sich wie folgt zusammen (FI.-% lt. gaschromatographischer Analyse, der Anteil an Toluol ist nicht berücksichtigt):

| | |
|---|---|
| Vorlaufkomponenten | 0,4 % |
| Eugenolmethylether | 2,6 % |
| Komponenten | 0,8 % |
| Isoeugenolmethylether (1,2-Dimethoxy-4-propenyl-1-benzol) | 9,9 % |
| Komponenten | 0,6 % |
| 2-Ethyl-2-(3,4-dimethoxy-phenyl)-acetaldehyd | 0,2 % |
| 2-Methyl-3-(3,4-dimethoxy-phenyl)-propionaldehyd | 5,1 % |
| 4-(3,4-Dimethoxy-phenyl)-butanal | 77,6 % |
| Nachlaufkomponenten | 2,8 % |

Die im Autoklav verbleibende Katalysatorphase kann erneut mit einem Gemisch aus Eugenolmethylether und Toluol versetzt und zur Reaktion gebracht werden.

Wie die Beispiele 1-3 belegen, führt das erfindungsgemäße Verfahren zu einem hohen Anteil an den entsprechend substituierten 4-Phenylbutanalen, bezogen auf den Gehalt an verzweigten substituierten 3-Phenylpropanalen.

## Patentansprüche

1. Verfahren zur Hydroformylierung von substituierten Allylbenzolen, **dadurch gekennzeichnet, dass** man Allylbenzole der allgemeinen Formel (I) 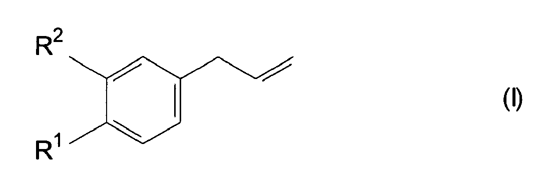 in der R¹ einen Hydroxyl- oder Alkoxyrest mit 1 bis 4 Kohlenstoffatomen bedeutet, R² für Wasserstoff, einen Hydroxyl- oder einen Alkoxyrest mit 1 bis 4 Kohlenstoffatomen steht, R¹ und R² gemeinsam eine Alkylendioxygruppe -[O-cH₂-O]ₙ- mit n gleich 1, 2 oder 3 bedeutet, in einem heterogenen Reaktionssystem unter Verwendung einer wässrigen Lösung von wasserlöslichen, sulfonierte Arylphosphine der allgemeinen Formel (II) 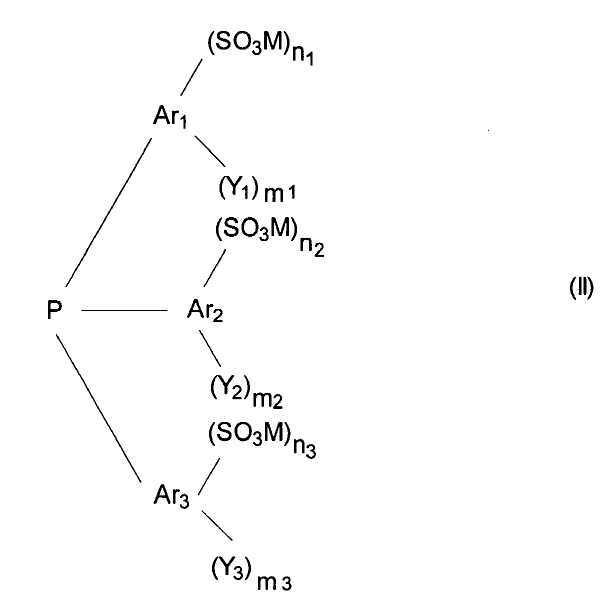 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR³R⁴ stehen, in der die Substituenten R³ und R⁴ gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist, in komplexer Bindung enthaltenden Rhodiumverbindungen bei Temperaturen von 80°C bis 140°C und Drücken von 0,5 MPa bis 10 MPa mit Synthesegas umsetzt.

2. Verfahren nach Anspruch 1, **dadurch gekennzeichnet, dass** man die Hydroformylierung bei Temperaturen von 100°C bis 130°C, vorzugsweise bei 110°C bis 130°C, durchführt.

3. Verfahren nach Anspruch 1 oder 2, dadruch **gekennzeichnet**, dass man die Hydroformylierung bei Drücken von 1 bis 6 MPa, vorzugsweise bei 1,5 bis 5 MPa, durchführt.

4. Verfahren nach einem oder mehreren der Ansprüche 1 bis 3, **dadurch gekennzeichnet, dass** die Rhodiumkonzentration 20 bis 1000 Gew.-ppm, vorzugsweise 50 bis 800 Gew.-ppm, und insbesondere 100 bis 600 Gew.-ppm, jeweils bezogen auf die wässrige Katalyatorlösung, beträgt.

5. Verfahren nach einem oder mehreren der Ansprüche 1 bis 4, **dadurch gekennzeichnet, dass** je mol Rhodium 10 bis 300 mol, insbesondere 50 bis 150 mol, Phosphor in Form der wasserlöslichen, sulfonierten Arylphosphine der allgemeinen Formel (II) verwendet werden.

6. Verfahren nach einem oder mehreren der Ansprüche 1 bis 5, **dadurch gekennzeichnet, dass** man als Allylbenzole 1-Hydroxy-2-methoxy-4-allylbenzol, 1-Methoxy-4-allylbenzol, 1,2-Dimethoxy-4-allylbenzol und 1,2-Methylendioxy-4-allylbenzol umsetzt.

7. Verfahren nach einem oder mehreren der Ansprüche 1 bis 6, **dadurch gekennzeichnet, dass** die nach Phasentrennung erhaltene wässrige Katalysatorlösung wieder in der Hydroformylierung eingesetzt wird.
